Europäisches Patentamt

(19) European Patent Office

Office européen des brevets

(11) **EP 0 735 860 B1**

(12) **FASCICULE DE BREVET EUROPEEN**

(45) Date de publication et mention
de la délivrance du brevet:
**06.05.1999 Bulletin 1999/18**

(21) Numéro de dépôt: **95903833.2**

(22) Date de dépôt: **14.12.1994**

(51) Int. Cl.⁶: $A61K\ 9/00$, $A61K\ 9/12$

(86) Numéro de dépôt international:
**PCT/FR94/01463**

(87) Numéro de publication internationale:
**WO 95/16433 (22.06.1995 Gazette 1995/26)**

(54) **NOUVELLES COMPOSITIONS POUR MOUSSES, NOTAMMENT MOUSSES RECTALES, ET MOUSSES AINSI OBTENUES**

NEUE SCHAUMZUSAMMENSETZUNGEN, INSBESONDERE ZUR REKTALEN ANWENDUNG, SOWIE DAVON ERHALTENE SCHÄUME

NOVEL FOAMING COMPOSITIONS, PARTICULARLY FOR RECTAL FOAMS, AND RESULTING FOAMS

(84) Etats contractants désignés:
**AT BE CH DE DK ES FR GB GR IE IT LI LU NL PT SE**

(30) Priorité: **14.12.1993 FR 9314973**

(43) Date de publication de la demande:
**09.10.1996 Bulletin 1996/41**

(73) Titulaire: **SCOPHYSA**
**75013 Paris (FR)**

(72) Inventeurs:
• **STAMM, André**
**F-67870 Griesheim (FR)**

• **CEPIK, Sibel**
**F-67400 Illkirsch (FR)**
• **WEHRLE, Pascal**
**F-67150 Erstein (FR)**

(74) Mandataire:
**Pochart, François et al**
**Cabinet Hirsch,**
**34, rue de Bassano**
**75008 Paris (FR)**

(56) Documents cités:
**EP-A- 0 395 329**     **EP-A- 0 533 938**
**FR-A- 2 647 344**

**Description**

L'invention concerne de nouvelles compositions pour mousses, notamment mousses rectales, et les mousses ainsi obtenues.

Certains médicaments nécessitent des administrations locales ou topiques. Ainsi, certains médicaments nécessitent une administration par voie orale ou par voie rectale, dans ce dernier cas dans le cadre d'un traitement de pathologies de l'ampoule rectale et autre (astringent, désinfectant), par exemple dans le cadre du traitement des inflammations rectales et hémorroïdaires, ou de la maladie de Crohn, etc. Dans ce contexte, il est opportun de rechercher une libération du principe actif au niveau de la zone à traiter. Un exemple d'un traitement rectal consiste en un traitement à base de mésalazine, ou ci-après 5-ASA, ou en un traitement à base d'hydrocortisone. Ainsi, on connaît des mousses rectales contenant de l'hydrocortisone, sous la marque Proctocort® ou Colifoam®.

Ces mousses sont de plusieurs types. Ainsi, on connaît les mousses aqueuses stables, les mousses non-aqueuses stables, les mousses aqueuses évanescentes (quick-breaking), et les mousses non-aqueuses évanescentes. Ces mousses peuvent être à base d'émulsions ou de suspensions du principe actif dans le support pharmaceutiquement acceptable; le gaz propulseur étant lui dispersé dans la phase liquide qui forme la phase dispersante.

Ces mousses présentent les avantages liés à ce type de formulation qui sont nombreux et indiqués partiellement ci-après. Les propriétés pharmacologiques des mousses sont supérieures à celles des suppositoires, présentant l'inconvénient d'une libération tardive et incomplète. Au contraire avec les mousses rectales, le principe actif est mis en contact avec la muqueuse sans temps de latence dû à la fusion ou à la dissolution de l'excipient. Les mousses rectales présentent aussi une grande facilité d'application, à la différence des solutions qui parfois ne sont pas conservées dans l'ampoule rectale, ce qui cause des désagréments à l'utilisateur. Les mousses rectales, étant moins rigides que les suppositoires, s'adaptent aisément aux contours et donc sont moins irritant. Le principe actif contenu dans la mousse, stockée dans un boîtier, est sous une forme stable, particulièrement si le composé chimique est sensible à la lumière et/ou à l'oxydation. Les mousses pressurisées permettent de masquer la goût des huiles alimentaires, ce qui est un avantage pour une administration par voie orale. Ces avantages justifient le surcoût lié à la fabrication de ces mousses, pourtant aisée, dû en partie aux coûts du boîtier, propulseur, valves, etc.

Cependant, les mousses, en particulier les mousses rectales présentent des inconvénients. Tout d'abord, ces mousses contiennent des gaz propulseurs à base de CFC, dont certains sont désormais proscrits. Des recherches dans le domaine des nouveaux gaz propulseurs permet de pallier cet inconvénient.

Le principal inconvénient des mousses rectales est leur densité faible, typiquement de l'ordre de 0,1 g/l, qui ne permet pas l'administration de quantités élevées de principe actif. Cette densité faible impose l'administration de grandes quantités de mousse, ce qui est problématique à cause du volume limité du rectum (entre environ 50 ml et 400 ml). Ce problème peut être résolu, au moins en partie, par la formulation de mousses fortement dosées en principe actif. On recherche donc des mousses qui permettent une concentration en principe actif élevée, de préférence supérieure à 30% en poids. Puisque les posologies classiques sont de 1 à quelques grammes de principe actif par jour, généralement en deux prises, la mousse doit avoir une densité relativement élevée pour ne pas représenter un volume trop important et provoquer un réflexe d'exonération. Par exemple, une densité de mousse d'au moins 0,1 g/ml, avec un taux de charge de 35% conduit à un volume d'environ 60 ml pour administrer 2 g de principe actif. Ces 60 ml, en comparaison du volume de 700 ml de l'ampoule rectale, ne provoque pas de réflexe d'exonération.

Selon le type d'application et de pathologie à traiter, on recherche une mousse ayant une expansion secondaire faible ou au contraire forte. On recherche une expansion secondaire forte si on souhaite que la mousse atteigne le colon descendant et ait alors une action systémique par passage au niveau du foie, ceci par exemple dans le cas d'un traitement antipyrétique à l'aide de paracétamol sous forme de mousse avec une action systémique. Au contraire, on recherche une expansion secondaire faible si on souhaite que la mousse reste circonscrite à un locus déterminé, dans le cas présent la zone inférieure du rectum pour par exemple le traitement des hémorroïdes.

La demande de brevet de Smith Kline & French Laboratories, EP-A-0 395 329, décrit des mousses contenant de 15 à 25% en poids de 5-ASA, et contenant des cires auto-émulsifiantes, du glycérol, des tensio-actifs, de la silice colloïdale et de l'eau, ainsi que des adjuvants classiques. Les particules de 5-ASA ont une dimension particulaire inférieure à 60 μm. La silice colloïdale est présente en une quantité inférieure à 1% en poids, la cire auto-émulsifiante étant présente en une quantité inférieure à 2% en poids. Cependant, ce type de formulation ne permet pas d'obtenir des taux de charge en principe actif qui soient élevés, i.e. supérieurs à 30% en poids.

La présente invention a donc pour objet de fournir une composition pour mousses, notamment celles destinées à être utilisées dans un traitement thérapeutique, qui permette une concentration en principe actif supérieure à 25%. Cette composition pour mousse est un concentré de mousse, c'est-à-dire le support pharmaceutique ajouté au gaz propulseur.

Ainsi, la présente invention fournit une composition pour mousse, comprenant, en poids par rapport au poids total de la composition:

(a) plus de 25% d'un principe actif sous forme de poudre;

(b) de 1 à 20% d'un tensio-actif; et

(c) la balance étant composée d'eau;

caractérisé en ce que la poudre dudit principe actif possède une dimension particulaire inférieure à 20 µm et une densité apparente comprise entre 250 et 450 g/dm$^3$.

Le terme de "balance étant composée d'eau" signifie que de l'eau est utilisée pour arriver à 100%, avec ou sans les divers additifs éventuellement utilisés, qui sont incorporés dans ce terme "balance constituée d'eau". Par ailleurs, ce terme recouvre aussi tout mélange principalement aqueux, et notamment les mélanges hydro-alcooliques, contenant comme alcool par exemple du glycerol, du propylèneglycol, du PEG-300 ou du PEG-400. Ce terme "balance d'eau" couvre aussi bien entendu les solution aqueuses tampon qui sont utilisées, comme par exemple une solution tampon phosphate, comprenant outre de l'eau purifiée, du phosphate monopotassique, de l'hydroxyde de sodium et de l'acide chlorhydrique en quantités suffisantes pour atteindre le pH recherché.

La demanderesse a en effet découvert avec surprise que des particules d'une dimension faible peuvent être présentes en quantités élevées dans les compositions. Par ailleurs, la composition selon l'invention permet de délivrer des doses précises de médicament, (exactes à +/- 10%), par passage au travers de valves doseuses (type LAB LABO).

Selon un mode de réalisation, la dimension particulaire est inférieure à 10 µm, avantageusement d'environ 5 à 6 µm. De telles dimensions inférieures à 20 µm peuvent être obtenues par tout procédé classique. Dans le cadre de l'invention, on utilise la technique dite de micronisation, en soumettant une poudre de dimension 25 µm, en deux passages dans un microniseur à jet d'air.

Cette faible dimension permet des concentrations en principe actif élevées, supérieures à 25%, ce qui était dans la majorité des cas la limite extrême avec des formulations selon l'art antérieur.

La demanderesse a aussi découvert avec surprise que des résultats particulièrement avantageux sont obtenus avec un principe actif présentant une densité apparente particulière.

Ainsi, le principe actif sous forme de poudre présente une densité apparente comprise entre 250 et 450 g/dm$^3$.

Avantageusement, le principe actif sous forme de poudre présente une densité apparente comprise entre 300 et 350 g/dm$^3$, par exemple d'environ 320 g/dm$^3$.

Selon un mode de réalisation, le principe actif représente de 30 à 50% en poids, avantageusement 35 à 45% en poids.

Le tensio-actif présent dans cette composition permet l'obtention d'une suspension sous la forme d'une mousse, le gaz se dispersant dans le liquide sous l'effet du tensio-actif. Ce terme tensio-actif couvre aussi les mélanges de tensio-actifs.

Selon un mode de réalisation, le tensio-actif représente de 5 à 10% en poids de la composition, avantageusement environ 7,5%.

Comme il a été précédemment écrit, on peut rechercher une expansion secondaire forte ou au contraire faible.

Lorsqu'on cherche une expansion secondaire élevée, on a recours aux tensio-actifs classiques et connus à cet effet, comme les cires auto-émulsifiantes telles que le lanol, etc. En fait, la quasi-majorité des tensio-actifs conduisent à une expansion plus ou moins forte.

Par contre, il n'existe jusqu'à présent aucune formulation qui offre une expansion secondaire faible. Selon une variante de l'invention, celle-ci fournit une mousse avec une expansion secondaire faible.

Selon cette variante, la présente invention fournit une composition dans laquelle le tensio-actif est un mélange de deux tensio-actifs, l'un étant un tensio-actif hydrophile présentant une valeur HLB supérieure à 10, l'autre étant un polyoxyalkylène.

Selon un mode de réalisation, le tensio-actif hydrophile présente une valeur HLB supérieure à 12, avantageusement supérieure à 15.

Un tensio-actif hydrophile apprécié est représenté par un polysorbate.

Selon un mode de réalisation, le tensio-actif à base de polyoxyalkylène est un poloxamère. Ce terme utilisé dans la présente invention désigne les polymères ayant une structure identique ou similaire à celle des polymères connus sous la dénomination commerciale Poloxamer$^®$. Les poloxamères sont des polymères contenant des unités polyoxyéthylène et polyoxypropylène, disposées de façon séquencée. Avantageusement, ce poloxamère présente un poids moléculaire supérieur à 5000.

Les poloxamères sont décrits en détail dans le livre Martindale (28°Ed., pages 375-376) et dans USP XXII/N.F.XVII (pages 1960-61). Un poloxamère préféré est le poloxamère 188 (Pluronic$^®$ F68).

Le rapport pondéral entre les deux tensio-actifs, hydrophile et polyoxyalkylène varie dans une large mesure. Cependant, on préfère les compositions dans laquelle le rapport pondéral tensio-actif hydrophile/polyoxyalkylène est compris entre 1 et 5, avantageusement environ 3.

Selon un mode de réalisation, le tensio-actif est un tensio-actif non-ionique. Les tensio-actifs non-ioniques sont moins irritants.

Selon un mode de réalisation, la présente composition comprend en outre un agent de mise en suspension.

Selon un mode de réalisation, la présente composition comprend en outre un agent muco-adhésif. Cet agent muco-adhésif assure un mise en contact optimale et une répartition uniforme de la mousse, par exemple dans l'ampoule rectale. Un agent muco-adhésif qui peut être employé dans la présente invention est la carboxyméthylcellulose de sodium, désignée ci-après par CMC sodique ou simplement CMC. La CMC possède en outre l'avantage d'être un agent de mise en suspension.

Par ailleurs, la présente composition peut en outre comprendre des additifs et adjuvants classiques. Ces additifs sont présents en des proportions classiques, n'interférant pas avec les propriétés physiques recherchées pour la mousse; des proportions classiques pour de tels additifs sont de 0,01 à 1 % en poids, par rapport au poids de la composition. De tels additifs sont les agents conservateurs et autres. Parmi les additifs on peut citer de façon non-limitative le benzoate de sodium, l'EDTA de sodium, le métabisulfite de sodium.

Le principe actif que peut contenir les présentes compositions est tout principe actif, insoluble dans l'eau ou au contraire soluble dans l'eau. Dans ce dernier cas, la solubilité n'est pas suffisante pour que tout le principe actif soit solubilisé, on se place alors à la limite de solubilité et le reste de principe actif est alors considéré comme insoluble. Des exemples de principe actifs qui peuvent être incorporés dans les présentes compositions et donc dans des mousses sont, à titre non-limitatif: fluticazone, beclomethazone, budesonide, ipsalazine, balsalazine, olsalazine, mésalazine (5-ASA), 4-ASA, salazopyrine, stéroïdes tels que hydrocortisone, prednisolone, et les anesthésiques locaux..

Selon un mode de réalisation, le principe actif est le 5-ASA.

Bien qu'il soit possible d'incorporer, dans les présentes compositions, des polyols tels que PEG 400, glycol, glycerol et autres, la présente invention permet aussi d'éviter l'emploi de ces polyols qui sont parfois irritants.

La présente invention se rapporte aussi aux mousses, c'est-à-dire au produit final directement utilisable par le patient. Ainsi, l'invention a aussi pour objet les mousses comprenant, pour 100 parts en poids:

de 80 à 95 parts de la composition selon l'invention; et
de 5 à 25 parts d'un gaz propulseur.

Ce gaz propulseur est tout gaz propulseur connu et approprié pour cette application. Le gaz propulseur est, de façon classique, approprié lorsqu'il conduit à une pression interne comprise entre 3 et 5 kg, et lorsqu'il représente de 5 à 25% de la mousse, de préférence de 10 à 15% en poids, par rapport au poids total de la mousse. Actuellement, un gaz propulseur connu est un mélange 12/114, 40/60 (vol/vol), la nomenclature étant celle connues des chlorofluorocarbones (CFC). Actuellement, de nouveaux gaz propulseurs sont en développement et remplaceront les CFC. Les compositions selon l'invention sont aussi appropriées pour former des mousses avec ces nouveaux gaz. En tant qu'exemple de gaz de substitution, on peut citer l'isobutane.

Les mousses selon la présente invention sont appropriées pour tout traitement thérapeutique nécessitant une application topique.

Selon un mode de réalisation, l'invention a pour objet des mousses pour une application rectale.

La présente invention se rapporte aussi au procédé de préparation des nouvelles compositions et mousses les contenant.

Ainsi, l'invention a aussi pour objet un procédé de préparation d'une composition selon l'invention, comprenant l'étape de dispersion dans l'eau de la poudre de principe actif.

Cette étape de dispersion est importante. En effet, le principe actif étant sous une forme pulvérulente avec une granulométrie très fine, son volume apparent est très élevé. Ainsi, le volume apparent de la poudre représente 5 fois, voire jusqu'à 10 fois, le volume d'eau dans lequel on disperse ledit principe actif. Il est donc nécessaire de disposer d'un disperseur efficace. Dans le présent cas, on utilise un homogénéiseur à filière du type "Gann Emulgor". Dans la présente description, les termes dispersion et homogénéisation seront employés de façon équivalente.

Selon un mode de réalisation, le procédé de préparation de la présente composition comprend les étapes suivantes:

(i) préparation d'un tampon;
(ii) ajout et mélange de l'agent muco-adhésif, s'il est présent;
(iii) ajout et mélange des additifs classiques, s'ils sont présents;
(iv) parallèlement aux étapes précédentes, préparation du tensio-actif;
(v) mélange du produit de l'étape (iii) et de l'étape (iv);
(vi) ajout et dispersion de la poudre de principe actif.

Selon une variante, le tensio-actif est préparé par mélange de deux tensio-actifs, l'un étant un tensio-actif hydrophile présentant une valeur HLB supérieure à 10, l'autre étant un polyoxyalkylène.

Les mousses finales sont obtenues classiquement par remplissage de boîtiers avec la présente composition, positionnement des valves desdits boîtiers, pressurisation par les propulseurs, et éventuellement conditionnement secondaire comme étiquetage, etc.

L'invention est maintenant décrite plus en détail dans la description et les exemples qui suivent, qui sont donnés à titre illustratif et nullement limitatif de la portée de l'invention, susceptible de nombreuses variantes aisément accessible à l'homme de l'art.

On donne ci-après les caractéristiques variables du concentré pour la mousse, c'est-à-dire la composition pour mousse. Dans ce qui suit, on désigne de façon équivalente le concentré et la composition selon l'invention.

Variables relatives à la composition:

Viscosité:
Un viscosimètre rotatif Haake (Viscosimeter Rotovisco RV 3) est utilisé pour déterminer la viscosité des concentrés ou compositions. Les tensions de cisaillement sont mesurées en fonction du gradient de vitesse de cisaillement. La température est stable (30+/-0,5°C) pour tous les essais.

Aspect de la composition:
L'aspect macroscopique du concentré est évalué de manière subjective, en tenant compte de l'homogénéité de la mousse, de la présence de grumeaux, de la viscosité, etc. Une note globale d'aspect (entre 0 et 1, 0 étant mauvais et 1 étant bon) est ainsi attribuée à chaque concentré. Cette évaluation est superflue pour les concentrés contenant du 5-ASA micronisé de dimension 6 $\mu$m, car tous les concentrés obtenus sont homogènes et obtiennent la note 1.

Sédimentation:
La sédimentation est étudiée dans un tube à essai après 48 heures. Un pourcentage de sédimentation S est calculé à partir du rapport de hauteur entre la phase séparée et la hauteur totale, comme l'indique la formule mathématique suivante.

$$S \% = 1 - \frac{\text{hauteur sédimentation}}{\text{hauteur totale}} \times 100$$

Variables relatives à la mousse:
On conditionne une mousse selon un protocole de pressurisation classique, explicité ci-après.

Aspect:
L'aspect de la mousse est note selon 4 critères différents:

- Bruit
- Homogénéité
- Expansion initiale
- Affaissement

La notation est subjective, et notée entre 0 et 1 pour chaque critère, 0 étant mauvais et 1 étant bon. La notation est effectuée comme suit:

| Emission bruyante: | 0 | Emission silencieuse: | 1 |
|---|---|---|---|
| Aspect non-homogène: | 0 | Aspect homogène: | 1 |
| Expansion initiale faible: | 0 | Expansion initiale forte: | 1 |
| Affaissement rapide: | 0 | Affaissement lent: | 1 |

La note totale (sur 4) prend en compte ces 4 valeurs.

Expansion secondaire:

Les expériences de mesure ne sont pas réalisées dans des éprouvettes graduées car l'expansion est trop rapide et intense pour permettre une mesure reproductible. Les formulations soumises au test ne présentent pas d'expansion secondaire. L'expansion est donc notée de façon qualitative avec l'aspect de la mousse.

Evaporation dans l'air ambiant:

Pour cette expérience, on utilise une boîte de Petri vide, dont le volume est connu. Cet espace est rempli avec de la mousse en excès, qui est arasée immédiatement après avec une spatule. L'ensemble est placé dans l'air ambiant, à température constante, et à l'abri des courants d'air. Le pourcentage de perte de poids en fonction du temps (rapporté au poids initial) est noté toutes les 5 mn pendant une heure. L'évaporation est la mesure après une heure, exprimée en g/100 g.

Etalement:

Pour cette mesure, 2 plaques de verre de 20 x 20 cm soigneusement dégraissée sont utilisées. Au milieu de la plaque inférieure, une quantité exactement pesée (1 g) de mousse est déposée. Puis la plaque supérieure, d'un poids de 126 g, est posée sur la première; la mousse emprisonnée entre les deux plaques s'étale. Le rayon, en cm, du cercle de mousse ainsi obtenu est noté après 3 mn. Cette mesure permet l'évaluation de la consistance.

Drainage:

On détermine le volume du liquide de drainage. Un entonnoir en verre, contenant une quantité prédéterminée de mousse, est posé sur une éprouvette graduée de 5 ml qui permet la lecture du volume de liquide perdu par drainage. Afin d'éviter l'évaporation de la mousse, une plaque de verre est posée sur l'entonnoir. La mesure est effectuée sur une prise d'essai de 5 g de mousse; le volume de liquide dans l'éprouvette est lu après 24 h.

Densité:

La mesure de la densité est réalisée à l'aide d'un entonnoir relié par un tuyau plastique à un bouton-poussoir qui peut d'adapter sur un boîtier pressurisé. Le poids de l'ensemble vide et le poids du volume d'eau que peut contenir cet ensemble (entonnoir-tube-poussoir) sont préalablement déterminés avec précision. Le remplissage du dispositif se fait en fixant le bouton-poussoir sur la valve du boîtier; ce système assure un remplissage de l'entonnoir uniforme et sans bulle d'air.

Exemples 1 à 33: (exemples comparatifs)

Dans ces exemples, le principe actif est remplacé par du talc, produit non-oxydable contrairement au 5-ASA, ayant sensiblement la même granulométrie et le même comportement physique que ce dernier. La dimension particulaire moyenne du talc est de 25 $\mu$m. On donne ci-après un tableau comparatif entre du talc et du 5-ASA, justifiant que leurs comportements rhéologiques sont comparables.

Tableau 1

|  | densité apparente | densité apparente tassée | rapport d'Hausner |
|---|---|---|---|
| Talc | 40 | 57,1 | 0,70 |
| 5-ASA | 61,9 | 77,6 | 0,80 |

Les compositions F1 à F33 sont données ci-après, par groupes, respectivement F1 à F11, F12 à F21, F22 à F33. Dans les tableaux qui suivent, les données sont en grammes de produit, pour un poids total de 100 g.

Les différentes formules contiennent deux véhicules principaux, l'eau et les dérivés glycoliques. L'introduction des constituants hydrophiles se fait dans l'eau et celle des constituants lipophiles se fait dans le glycol ou ses analogues. Le mode de préparation est le suivant:

- dissolution dans la phase aqueuse des sels solubles; métabisulfite de sodium, parabène, édétate sodique et ensuite dispersion de la silice colloïdale;
- incorporation dans la phase glycolique du polysorbate 20 et 80;
- chauffage séparé des deux solutions vers 65°C puis dispersion de la poudre dans le polyol à l'aide d'un agitateur magnétique;
- mélange à chaud des deux solutions (la solution aqueuse est versée dans la solution glycolique) sous agitation jusqu'à refroidissement complet;
- conditionnement de la suspension en flacons, la solution étant versée dans le boîtier puis la valve est sertie et le gaz est introduit au travers de la valve; le remplissage de gaz se fait par une table de pressurisation Cel 3® (Coster S.A.).

Le même protocole de préparation est appliqué sans exception à toutes les formules, mêmes à celles ne contenant pas l'un ou l'autre des composants.

Pour ces 33 exemples selon l'art antérieur, on détermine les caractéristiques énoncées ci-avant:

- Aspect de la suspension: homogène et stable et suffisamment visqueux pour permettre la pressurisation;
- Pour les mousses: une expansion nulle, légère ou forte, la consistance (liquide ou ferme), le comportement à l'éjection (bon comportement ou sortie bruyante).

On remarque que certaines compositions ne sont pas adaptées, notamment F1 à F16, F19, F21 à F23, F25 à F30, et F32, puisque ces compositions ne permettent pas une pressurisation. Les concentrés qui sont pressurisés pour conduire à une mousse sont donc F17, F18, F20, F24, F31 et F33. Toutes les mousses obtenues à partir de ces composés, à l'exception de celle obtenue à partir de la composition F33, présentent une expansion forte et sont liquides. Seule la mousse obtenue à partir de la composition F33 est compacte et légèrement expansive.

Par ailleurs, il convient de noter que ces formulations ne permettent un taux de charge que de 20%.

Exemples 34 à 41: (comparatifs)

On prépare maintenant des formulations contenant un principe actif. Il s'agit ici de 5-ASA, dont les caractéristiques principales ont été données ci-avant. Le 5-ASA est obtenu chez Nobel Chemicals. La densité du 5-ASA est d'environ 319 g/dm$^3$. Le protocole de préparation des mousses est identique à celui utilisé pour préparer les formulations F1 à F33, à l'exception du fait que l'on utilise un appareil Polytron® (à la vitesse 3 sur le cadran pendant 2 mn afin d'obtenir une masse homogène.

Les compositions sont consignées dans le tableau 3 ci-après.

Les caractéristiques de ces mousses sont données ci-après:

Les mousses obtenues à partir des compositions F34 et F35 sont peu expansives, d'aspect compact et solides. Les mousses obtenues à partir des compositions F36 et F37 sont aussi d'aspect compact et solides, mais leur sortie est difficile car le concentré est très visqueux, et elles présentent de plus une expansion forte. Les mousses obtenues à partir des compositions F38 et F40 ont une forte expansion. La mousse obtenue à partir de la composition F39 est compacte, stable et peu expansive. La mousse obtenue à partir de la composition F41 est peu expansive mais présente des grosses bulles et est légèrement liquide.

Exemples A1 à A12: (comparatifs)

On prépare des concentrés comme précédemment, puis des mousses à partir de ceux-ci. Les protocoles utilisés sont ceux exposés ci-avant. Les résultats sont consignés dans le tableau 4 ci-après.

Les caractéristiques de ces mousses sont résumées ci-après:

Les concentrés A1 à A5 et A9 ne permettent pas la pressurisation, soit par défaut de stabilité du concentré (celui-ci sédimente), soit à cause d'une viscosité élevée.

Les concentrés A6, A7, A8, A11 conduisent à des mousses liquides.

La mousse obtenue à partir du concentré A10 ne présente pas un bon comportement à la sortie.

Exemples OP1 à OP23:

On prépare les formulations OP1 à OP23 comme précédemment. Les exemples OP1, OP2, OP5 et OP6 sont des exemples comparatifs, la dimension des particules étant de 25 µm, tandis que la dimension des particules pour les exemples OP3, OP4, et OP7 à OP23 est d'environ 6 µm.

Les compositions et caractéristiques des formulations OP1 à OP8 sont résumées dans le tableau 5 ci-après. Le gaz propulseur est un mélange F114/F12 selon un rapport en poids variable et représentant de 15 à 20% en poids de la mousse finale. Les pressions respectives obtenues avec ces mélanges sont indiquées dans le tableau 5.

Dans les formulations OP9 à OP23, le PEG 400 est supprimé, le diamètre des particules est fixé à environ 6 µm, la pression du boîtier est maintenue à environ 5 atm. Le rapport pondéral entre le polysorbate 80 et le pluronic®F68 est maintenu à environ 3.

Les compositions et les caractéristiques des formulations OP9 à OP23 sont résumées dans le tableau 6 ci-après.

On utilise deux critères, stabilité de la suspension et aspect de la mousse. Un panel d'experts est utilisé pour la notation; on reporte dans le tableau la moyenne des notes données par les juges. La note d'aspect est donnée sur 4, et non plus sur 1. Cette note prend en compte les critères suivants:

- bruit: pendant l'éjection de la mousse du boîtier l'absence de bruit est notée 1; la présence de bruit est notée 0;
- homogénéité: cette caractéristique de la mousse est jugée de manière macroscopique par la comparaison de la taille des bulles entre elles, la mousse devant présenter une structure aussi régulière que possible pour obtenir la note 1; inversement une note de 0 traduit une mauvaise homogénéité;
- expansion: une mousse aérée, ferme et onctueuse est notée 1, une mousse peu expansive, liquide est sanctionnée par la note 0;
- affaissement: une tenue correcte est notée 1 tandis qu'un affaissement marqué après 2 mns est noté 0.

On remarque que les mousses selon l'invention permettent un taux de charge élevé, tout en assurant de bonnes propriétés à la mousse.

Exemple S1:

On prépare la composition suivante, tout d'abord pour des quantités à l'échelle de laboratoire puis pour des quantités à l'échelle de 1 kg de composition. Les proportions sont données dans le tableau 7 ci-dessous.

Tableau 7

| Composant | S1 |
|---|---|
| 5-ASA | 40,58 |
| CMC sodique | 0,9 |
| polysorbate 80 | 5,63 |
| Pluronic F68 | 1,88 |
| benzoate de sodium | 0,2 |
| EDTA sodique | 0,1 |
| métabisulfite de sodium | 0,13 |
| tampon pH 4,5 | 50,58 |

Le mode opératoire utilisé pour la préparation à l'échelle du laboratoire est le suivant:

- préparation du tampon phosphate pH 4,5 (selon PF X°Ed. p.VII.1.3. Solutions Tampons);
- pesée de la carboxyméthylcellulose de sodium (CMC sodique)
- incorporation de la CMC sodique dans le tampon, la solution est mise sous agitation par barreau magnétique pendant 60 mn;
- pesée des conservateurs; ils sont pesés séparément et recueillis dans une coupelle en inox;
- ajout des conservateurs dans la solution de CMC sodique, une fois que cette dernière est complètement homogène; (il est conseillé de ne pas ajouter les conservateurs avant la CMC sodique, car cela provoquerait une augmentation du temps de gonflement de la solution.);
- pesée des agents tensio-actifs, puis mise sous agitation par barreau magnétique dans un bécher;
- ajout des agents tensio-actifs à la solution quand les conservateurs ont été correctement dissous, avec agitation pendant 15 mn;
- pesée du 5-ASA et incorporation par petites quantités dans la solution obtenue, le mélange devenant de plus en plus pâteux, on continue à le travailler avec une spatule:
- passage du concentré obtenu dans un homogénéiseur à filière "Gann Emulgor", on prend soin de ne pas trop serrer la filière de manière à ne pas briser la structure de la CMC qui confère une certaine stabilité à la préparation; l'homogénéisation se fait en un seul passage;
- remplissage des flacons à raison de 70 g par flacon;
- pose et sertissage de la valve;
- pressurisation du boîtier avec un mélange fréon F12/F114 (selon un rapport pondéral de 40/60, le gaz représentant environ 15% du poids final de la préparation);
- emballage du flacon en verre dans des feuilles d'aluminium pour mettre la solution à l'abri de la lumière.

Le mode opératoire utilisé pour la préparation à l'échelle de 1 kg et plus est le suivant:

- préparation du tampon phosphate pH 4,5 (selon PF X°Ed. p.VII.1.3. Solutions Tampons);
- pesée de la carboxyméthylcellulose de sodium (CMC sodique)
- incorporation de la CMC sodique dans le tampon, la solution est mise sous agitation, à 3500 tpm dans un mélangeur haute vitesse type "Stephan" pendant 45 mn;
- pesée des conservateurs; ils sont pesés séparément et recueillis dans une coupelle en inox;
- ajout des conservateurs dans la solution de CMC sodique, une fois que cette dernière est complètement homogène; (il est conseillé de ne pas ajouter les conservateurs avant la CMC sodique, car cela provoquerait une augmentation du temps de gonflement de la solution.);
- pesée des agents tensio-actifs, puis mise sous agitation par barreau magnétique dans un bécher;
- ajout des agents tensio-actifs à la solution quand les conservateurs ont été correctement dissous, avec agitation pendant 15 mn;
- pesée du 5-ASA et incorporation par petites quantités dans la solution obtenue, le mélange devenant de plus en plus pâteux, on continue à le travailler avec une spatule:
- passage du concentré obtenu dans un homogénéiseur à filière "Gann Emulgor", on prend soin de ne pas trop serrer la filière de manière à ne pas briser la structure de la CMC qui confère une certaine stabilité à la préparation; l'homogénéisation se fait en un seul passage;
- la suite du mode opératoire est identique à celui exposé ci-avant.

Exemple S2:

On procède comme dans l'exemple précédent, mais avec la formulation et le gaz indiqué dans le tableau 8 ci-après. Cette formulation, avec l'isobutane est appropriée pour respecter de nouvelles réglementations qui interdisent l'emploi de CFC en tant que gaz propulseur.

Dans le tableau 8 qui suit, on indique les porportions centésimales en poids, sauf pour l'adjuvant de pressurisation ( en bar).

Tableau 8

| Composant | fonction | % en poids |
|---|---|---|
| 5-ASA | principe actif | 38,35 |
| Carboxyméthylcellulose sodique D | viscosifiant | 0,39 |
| Polysorbate 80 | tensio-actif | 5,32 |
| Poloxamer 188 | tensio-actif | 1,78 |
| Benzoate de sodium | conservateur | 0,19 |
| EDTA sodique | complexant | 0,095 |
| Disulfite de sodium | antioxydant | 0,123 |
| Tampon phosphate pH 4,5 | solvant | 48,27 |
| Isobutane | gaz propulseur | 5,5 |
| Azote | adjuvant de pressurisation | 5,5 bar |

Exemple EC1:

On prépare une composition selon l'exemple 2 de la demande de brevet européen EP-A-0 395 329 au nom de Smith Kline and French Laboratories Ltd., à l'exclusion du fait que le 5-ASA est utilisé à hauteur de 30% en poids dans la formulation.

Dans le premier cas, la dimension particulaire est d'environ 25$\mu$m, tandis que la densité apparente est comprise entre 300 et 350 g/dm$^3$. Pour obtenir un concentré homogène il est nécessaire de recourir à un homogénéiseur Polytron; cependant la mousse produite est d'une qualité très mauvaise: le concentré étant très épais, il faut agiter très vigoureusement le boîtier avant utilisation pour permettre une sortie, imparfaite, de la mousse.

Dans le second cas, la dimension particulaire est d'environ 25$\mu$m, tandis que la densité apparente est inférieure à 250 g/dm$^3$. La fabrication est alors impossible.

Exemple EC2:

On procède comme dans l'exemple EC1, mais cette fois le taux de charge en 5-ASA est de 35% en poids. La dimension des particules de 5-ASA est de 25 $\mu$m. Quelle que soit la densité, il est impossible d'obtenir une formulation.

Ces exemples EC1 et EC2 montrent que l'enseignement contenu dans la demande de brevet EP-A-0 395 329 n'est pas suffisant pour conduire à l'obtention de formulation contenant plus de 25% en poids de 5-ASA. La présente invention, au contraire, permet des taux de charge nettement supérieurs à ce seuil de 25%.

La présente invention n'est pas limitée aux modes de réalisation décrits mais est susceptible de nombreuses variantes aisément accessibles à l'homme de l'art.

Tableau 2

| | F1 | F2 | F3 | F4 | F5 | F6 | F7 | F8 | F9 | F10 | F11 |
|---|---|---|---|---|---|---|---|---|---|---|---|
| talc | 20 | 20 | 10 | 10 | 10 | 10 | 10 | 10 | 10 | 10 | 10 |
| cire auto-émulsifiante lanol CTO | 0,3 | 0,3 | 0,3 | 0,3 | 0,3 | 0,3 | 0,3 | 0,3 | 0,3 | 0,3 | 0,3 |
| polysorbate 20 | 1 | 6 | 6 | 1 | 0 | 6 | 0 | 6 | 2 | 1 | 2 |
| ester de sorbitane 80 | 6 | 1 | 6 | 1 | 6 | 0 | 6 | 0 | 1 | 2 | 1 |
| propylène glycol | 50 | 55 | 50 | 50 | 55 | 55 | | | | | 30,35 |
| glycérol | | | | | | | | | | | 30,35 |
| PEG 400 | | | | | | | 55 | 55 | 60,7 | 60,7 | |
| méthyl parabène | 0,2 | 0,2 | 0,2 | 0,2 | 0,2 | 0,2 | 0,2 | 0,2 | 0,2 | 0,2 | 0,2 |
| propyl parabène | 0,04 | 0,04 | 0,04 | 0,04 | 0,04 | 0,04 | 0,04 | 0,04 | 0,04 | 0,04 | 0,04 |
| silice colloïdale | 0,4 | 0,8 | 1 | | 0,2 | 0,2 | 0,2 | 0,2 | 0,2 | 0,2 | 0,2 |
| EDTA sodique | 0,1 | 0,1 | 0,1 | 0,1 | 0,1 | 0,1 | 0,1 | 0,1 | 0,1 | 0,1 | 0,1 |
| métabisulfite sodique | 3 | 3 | 3 | 3 | 0,3 | 0,3 | 0,3 | 0,3 | 0,3 | 0,3 | 0,3 |
| tampon q.s.p. 100 g | 18,96 | 13,96 | 23,36 | 33,36 | 25,16 | 25,16 | 25,16 | 25,16 | 25,16 | 25,16 | 25,16 |

Tableau 2 (suite)

| | F12 | F13 | F14 | F15 | F16 | F17 | F18 | F19 | F20 | F21 |
|---|---|---|---|---|---|---|---|---|---|---|
| talc | 10 | 10 | 10 | 10 | 10 | 10 | 10 | 10 | 10 | 10 |
| cire auto-émulsifiante lanol CTO | 0,3 | 0,3 | 0,5 | 1,0 | 1,5 | 2,0 | 1,5 | 1,5 | 1,0 | 1,5 |
| polysorbate 20 | 1 | 2 | 2 | 2 | 2 | 2 | 2 | 2 | 2 | 2 |
| ester de sorbitane 80 | 2 | 1 | 1 | 1 | 1 | 1 | 1 | 1 | 1 | 1 |
| propylène glycol | 30,35 | 30,35 | | | | | | | | |
| glycérol | 30,35 | 30,35 | | | | | | | | |
| PEG 400 | | | 60 | 60 | 60 | 60 | 50 | 70 | 50 | 42,5 |
| méthyl parabène | 0,2 | 0,2 | | | | | | | | |
| propyl parabène | 0,04 | 0,04 | | | | | | | | |
| silice colloïdale | 0,2 | 0,2 | | | | | | | | |
| EDTA sodique | 0,1 | 0,1 | | | | | | | | |
| métabisulfite sodique | 0,3 | 0,3 | | | | | | | | |
| tampon q.s.p. 100 g | 25,16 | 25,16 | 25 | 25 | 25 | 25 | 35 | 15 | 35 | 42,5 |

Tableau 2 (suite)

| | F22 | F23 | F24 | F25 | F26 | F27 | F28 | F29 | F30 | F31 | F32 | F33 |
|---|---|---|---|---|---|---|---|---|---|---|---|---|
| talc | 10 | 10 | 10 | 10 | 10 | 10 | 10 | 10 | 10 | 10 | 10 | 10 |
| cire auto-émulsifiante lanol CTO | 1 | 1 | 1 | 1 | 1 | 1 | 1 | 1 | 1 | 1 | 1 | 1 |
| polysorbate 20 | | | | | | | | | | | | |
| polysorbate 80 | 0,5 | 1,5 | 2,5 | 3,5 | 4,5 | 5,5 | 6,5 | 7,5 | 8,5 | 9,5 | 10,5 | 11,5 |
| propylène glycol | | | | | | | | | | | | |
| glycérol | | | | | | | | | | | | |
| PEG 400 | 50 | 50 | 50 | 50 | 50 | 50 | 50 | 50 | 50 | 50 | 50 | 50 |
| méthyl parabène | | | | | | | | | | | | |
| propyl parabène | | | | | | | | | | | | |
| silice colloïdale | | | | | | | | | | | | |
| EDTA sodique | | | | | | | | | | | | |
| métabisulfite sodique | | | | | | | | | | | | |
| tampon q.s.p. 100 g | 35 | 35 | 35 | 35 | 35 | 35 | 35 | 35 | 35 | 35 | 35 | 35 |

Tableau 3

| | F34 | F35 | F36 | F37 | F38 | F39 | F40 | F41 |
|---|---|---|---|---|---|---|---|---|
| 5-ASA | 10 | 15 | 20 | 25 | 25 | 20 | 25 | 25 |
| cire auto-émulsifiante lanol CTO | 0,92 | 0,89 | 0,79 | 0,69 | 0,69 | 0,79 | 0,69 | 1,24 |
| polysorbate 20 | | | | | | | | 1,65 |
| polysorbate 80 | 10,52 | 10,02 | 9,52 | 8,82 | 8,82 | 9,52 | 8,82 | |
| ester de sorbitane 80 | | | | | | | | 0,83 |
| propylène glycol | | | | | | | | |
| glycérol | | | | | | | | |
| PEG 400 | 45,85 | 43,18 | 40,58 | 38,18 | 37,88 | 40,28 | 37,58 | 35,12 |
| méthyl parabène | 0,2 | 0,2 | 0,2 | 0,2 | 0,2 | 0,2 | 0,2 | 0,2 |
| propyl parabène | 0,04 | 0,04 | 0,04 | 0,04 | 0,04 | 0,04 | 0,04 | 0,04 |
| silice colloïdale | | | | | 0,4 | 0,4 | 0,4 | 0,4 |
| EDTA sodique | 0,1 | 0,1 | 0,1 | 0,1 | 0,1 | 0,1 | 0,1 | 0,1 |
| métabisulfite sodique | 0,3 | 0,3 | 0,3 | 0,3 | 0,3 | 0,3 | 0,3 | 0,3 |
| tampon q.s.p. 100 g | 32,07 | 30,27 | 28,47 | 26,67 | 26,57 | 28,37 | 26,27 | 35,12 |

Tableau 4

| | A1 | A2 | A3 | A4 | A5 | A6 | A7 | A8 | A9 | A10 | A11 | A12 |
|---|---|---|---|---|---|---|---|---|---|---|---|---|
| 5-ASA | 35 | 35 | 35 | 35 | 35 | 35 | 35 | 35 | 35 | 35 | 35 | 35 |
| cire auto-émulsi-fiante lanol CTO | | | | | | | | | 2 | | | 1 |
| lécithine de soja | 1 | 1 | 2 | 2 | 2 | 1 | 1 | | | | | |
| polysorbate 20 | | | | | | | | 1,65 | | | | |
| polysorbate 80 | | | | 6 | 6 | | | | | 5 | | |
| ester de sorbitane 80 | | | | 4 | 4 | | | | | | 5 | |
| Pluronic F68 | | | | | | | | 1 | | 1 | 1 | |
| propylène glycol | | | | | | | | | | | | |
| glycérol | | | | | | | | | | | | |
| PEG 400 | | | | 10 | 10 | 10 | 10 | 10 | 10 | 10 | 10 | 10 |
| CMC | | 1 | 2 | 2 | 1 | 1 | 1 | 1 | 1 | 1 | 1 | 1 |
| méthyl parabène | 0,2 | 0,2 | 0,2 | 0,2 | 0,2 | 0,2 | 0,2 | 0,2 | | | | |
| propyl parabène | 0,04 | 0,04 | 0,04 | 0,04 | 0,04 | 0,04 | 0,04 | 0,04 | | | | |
| silice colloïdale | | | | | | | | | | | | |
| EDTA sodique | | | | | | | | | | | | |
| métabisulfite Na | | | | | | | | | | • | | |
| tampon qsp 100 g | 64 | 62 | 61 | 41 | 42 | 53 | 53 | 53 | 52 | 48 | 48 | 53 |
| F114/F12 | 60/40 | 60/40 | 60/40 | 60/40 | 60/40 | 100/0 | 0/100 | 60/40 | 60/40 | 60/40 | 60/40 | 60/40 |
| % en poids du gaz | 20 | 20 | 20 | 20 | 20 | 15 | 15 | 15 | 15 | 15 | 15 | 15 |

Tableau 5

| | OP1 | OP2 | OP3 | OP4 | OP5 | OP6 | OP7 | OP8 |
|---|---|---|---|---|---|---|---|---|
| 5-ASA | 40 | 30 | 40 | 30 | 40 | 30 | 40 | 30 |
| polysorbate 80 | 8,57 | 7,5 | 7,5 | 8,57 | 7,5 | 8,57 | 8,57 | 7,5 |
| Pluronic F68 | 1,43 | 2,5 | 2,5 | 1,43 | 2,5 | 1,43 | 1,43 | 2,5 |
| CMC | 1,5 | 0,5 | 0,5 | 1,5 | 1,5 | 0,5 | 0,5 | 1,5 |
| PEG 400 | 4,85 | 5,95 | 4,95 | 5,85 | | | | |
| tampon q.s.p. 100 g | 43,65 | 53,55 | 44,55 | 52,65 | 48,5 | 59,5 | 49,5 | 58,5 |
| Concentré | | | | | | | | |
| viscosité (mPa.s) | 6353 | 237 | 619 | 1581 | 5156 | 206 | 413 | 1485 |
| sédimentation en % | 0 | 4,6 | 0 | 0 | 0 | 1 | 0 | 0 |
| aspect (noté sur 1) | 0,25 | 1 | 1 | 1 | 0,5 | 0,75 | 1 | 1 |
| Mousse | | | | | | | | |
| pression boîtier (atm) | 5 | 3,2 | 5 | 3,5 | 2,6 | 4,6 | 3,5 | 5 |
| densité (g/ml) | 0,12 | 0,03 | 0,11 | 0,06 | 0,08 | 0,05 | 0,05 | 0,11 |
| expansion secondaire | | | | | | | | |
| étalement après 3 mn (cm) | 4,5 | 8 | 7,8 | 6,5 | 6 | 10 | 8 | 6,25 |
| évaporation en 60 mn | 8 | 18,2 | 9 | 22,1 | 13,6 | 45,6 | 25,6 | 15,1 |
| drainage en ml sur 24 h | 0,3 | 0,85 | 0 | 0,1 | 0,6 | 1,35 | 0,1 | 0,1 |
| aspect (noté sur 1) | 0,125 | 1 | 0,125 | 0,75 | 0,25 | 0,5 | 0,5 | 0,25 |

Tableau 6

| | OP9 | OP10 | OP11 | OP12 | OP13 | OP14 | OP15 | OP16 |
|---|---|---|---|---|---|---|---|---|
| 5-ASA | 30 | 40 | 30 | 40 | 30 | 40 | 30 | 40 |
| polysorbate 80 | 3,75 | 3,75 | 3,75 | 3,75 | 7,5 | 7,5 | 7,5 | 7,5 |
| Pluronic F68 | 1,25 | 1,25 | 1,25 | 1,25 | 2,5 | 2,5 | 2,5 | 2,5 |
| CMC | 0,5 | 0,5 | 1,5 | 1,5 | 0,5 | 0,5 | 1,5 | 1,5 |
| tampon qsp 100 g | 64,5 | 54,5 | 63,5 | 53,5 | 59,5 | 49,5 | 58,5 | 48,5 |
| Concentré | | | | | | | | |
| viscosité (mPa.s) | 94 | 291 | 766 | 2733 | 139 | 486 | 917 | 4263 |
| sédimentation (%) | 3,45 | 2,33 | 0,66 | 0 | 3,53 | 2,65 | 0,71 | 0 |
| aspect (note sur 1) | | | | | | | | |
| Mousse | | | | | | | | |
| densité (g/ml) | 0,04 | 0,06 | 0,06 | 0,12 | 0,05 | 0,07 | 0,12 | 0,29 |
| expansion secondaire | | | | | | | | |
| étalement après 3 mn (cm) | 9,1 | 7,2 | 6 | 44,4 | 8,4 | 6,7 | 5,5 | 3,8 |
| évaporation en 60 mn (g/100g) | 34,17 | 40,12 | 36,03 | 25,03 | 37,91 | 29,35 | 23,28 | 15,48 |
| drainage en ml sur 24 h | ? | ? | ? | ? | ? | | | |
| aspect (note sur 1) | ? | ? | ? | ? | ? | ? | ? | ? |

EP 0 735 860 B1

Tableau 6 (suite)

|  | OP17 | OP18 | OP19 | OP20 | OP21 | OP22 | OP23 |
|---|---|---|---|---|---|---|---|
| 5-ASA | 35 | 41,08 | 28,93 | 35 | 35 | 35 | 35 |
| polysorbate 80 | 5,63 | 5,63 | 5,63 | 5,63 | 5,63 | 7,91 | 3,35 |
| Pluronic F68 | 1,88 | 1,88 | 1,88 | 1,88 | 1,88 | 2,64 | 1,12 |
| CMC | 1 | 1 | 1 | 1,61 | 0,39 | 1 | 1 |
| tampon qsp 100 g | 56,5 | 50,41 | 62,56 | 55,88 | 57,10 | 53,45 | 59,53 |
| Concentré |  |  |  |  |  |  |  |
| viscosité (mPa.s) | 626 | 2178 | 343 | 2159 | 169 | 839 | 531 |
| sédimentation (%) | 0,86 | 0 | 3,77 | 0 | 3,57 | 0,82 | 2 |
| aspect (note sur 1) |  |  |  |  |  |  |  |
| Mousse |  |  |  |  |  |  |  |
| densité (g/ml) | 0,10 | 0,10 | 0,05 | 0,08 | 0,05 | 0,07 | 0,06 |
| expansion secondaire |  |  |  |  |  |  |  |
| étalement après 3 mn (cm) | 6 | 5,29 | 7,03 | 5,06 | 7,58 | 5,87 | 6,32 |
| évaporation en 60 mn (g/100g) | 28,48 | 17,61 | 27,91 | 19,83 | 32,77 | 22,35 | 27,50 |
| drainage en ml sur 24 h |  |  |  |  |  |  |  |
| aspect (note sur 1) |  |  |  |  |  |  |  |

## Revendications

1. Composition pour mousse, comprenant, en poids par rapport au poids total de la composition:

   (a) plus de 25% d'un principe actif sous forme de poudre;
   (b) de 1 à 20% d'un tensio-actif; et
   (c) la balance étant composée d'eau;

caractérisé en ce que la poudre dudit principe actif possède une dimension particulaire inférieure à 20 μm et une densité apparente comprise entre 250 et 450 g/dm$^3$.

2. Composition selon la revendication 1, dans laquelle la dimension particulaire est inférieure à 10 μm.

3. Composition selon la revendication 1 ou 2, dans laquelle le principe actif sous forme de poudre présente une densité apparente comprise entre 300 et 350 g/dm$^3$.

4. Composition selon l'une quelconque des revendications 1 à 3, dans laquelle le principe actif représente de 30 à 50% en poids.

5. Composition selon la revendication 4, dans laquelle le principe actif représente de 35 à 45% en poids.

6. Composition selon l'une quelconque des revendications 1 à 5, dans laquelle le tensio-actif représente de 5 à 10%.

7. Composition selon l'une quelconque des revendications 1 à 6, dans laquelle le tensio-actif est un mélange de deux tensio-actifs, l'un étant un tensio-actif hydrophile présentant une valeur HLB supérieure à 10, l'autre étant un polyoxyalkylène.

8. Composition selon la revendication 7, dans laquelle le tensio-actif hydrophile présente une valeur HLB supérieure à 12.

9. Composition selon la revendication 7 ou 8, dans laquelle le tensio-actif hydrophile est du polysorbate.

10. Composition selon l'une quelconque des revendications 8 à 9, dans laquelle le tensio-actif à base de polyoxyalkylène est un poloxamère.

11. Composition selon l'une quelconque des revendications 7 à 10, dans laquelle le tensio-actif à base de polyoxyalkylène présente un poids moléculaire supérieur à 5000.

12. Composition selon l'une quelconque des revendications 7 à 11, dans laquelle le rapport pondéral tensio-actif hydrophile/polyoxyalkylène est compris entre 1 et 5.

13. Composition selon l'une quelconque des revendications 1 à 12, dans laquelle le tensio-actif est un tensio-actif non-ionique.

14. Composition selon l'une quelconque des revendications 1 à 13, comprenant en outre un agent muco-adhésif.

15. Composition selon l'une quelconque des revendications 1 à 14, comprenant en outre des additifs et adjuvants classiques.

16. Composition selon l'une quelconque des revendications 1 à 15, dans laquelle le principe actif est de la mésalazine.

17. Mousse comprenant, pour 100 parts en poids:

de 80 à 95 parts de la composition selon l'une quelconque des revendications 1 à 16; et
de 5 à 25 parts d'un gaz propulseur.

18. Mousse selon la revendication 17, pour une application rectale.

19. Procédé de préparation d'une composition selon l'une quelconque des revendications 1 à 16, comprenant l'étape de dispersion dans l'eau de la poudre de principe actif.

20. Procédé selon la revendication 19, comprenant les étapes suivantes:

(i) préparation d'un tampon;
(ii) ajout et mélange de l'agent muco-adhésif, s'il est présent;
(iii) ajout et mélange des additifs classiques, s'ils sont présents;

(iv) parallèlement aux étapes précédentes, préparation du tensio-actif;

(v) mélange du produit de l'étape (iii) et de l'étape (iv);

(vi) ajout et dispersion de la poudre de principe actif.

21. Procédé selon la revendication 20, dans lequel le tensio-actif est préparé par mélange de deux tensio-actifs, l'un étant un tensio-actif hydrophile présentant une valeur HLB supérieure à 10, l'autre étant un polyoxyalkylène.

**Claims**

1. A composition for a foam, comprising, by weight based on the total weight of said composition:

   (a) more than 25% of an active ingredient in powder form;
   (b) from 1 to 20% of a surfactant; and
   (c) the balance being composed of water;

   characterized in that the powder of said active ingredient has a particle size below 20 $\mu$m and a bulk density comprised between 250 and 450 g/dm$^3$.

2. The composition according to claim 1, in which said particle size is below 10 $\mu$m.

3. The composition according to claim 1 or 2, in which the active ingredient in powder form has a bulk density comprised between 300 and 350 g/dm$^3$.

4. The composition according to any one of claims 1 to 3, in which the active ingredient makes up from 30 to 50% by weight of said composition.

5. The composition according to claim 4, in which the active ingredient makes up from 35 to 45% by weight .

6. The composition according to any one of claims 1 to 5 in which said surfactant makes up from 5 to 10%.

7. The composition according to any one of claims 1 to 6, in which said surfactant is a mixture of two surfactants, one of said surfactants being a hydrophilic surfactant having an HLB value above 10, the other being a polyoxyalkylene.

8. The composition according to claim 7, in which said hydrophilic surfactant has an HLB value above 12.

9. The composition according to claim 7 or 8, in which said hydrophilic surfactant is polysorbate.

10. The composition according to any one of claims 8 to 9, in which said polyoxyalkylene-based surfactant is a poloxamer.

11. The composition according to any one of claims 8 to 10, in which said polyoxyalkylene-based surfactant has a molecular weight above 5,000.

12. The composition according to any one of claims 8 to 11, in which the hydrophilic/polyoxyalkene surfactant weight ratio is comprised between 1 and 5.

13. The composition according to any one of claims 1 to 12, in which said surfactant is a non-ionic surfactant.

14. The composition according to any one of claims 1 to 13, further comprising a muco-adhesive agent.

15. The composition according to any one of claims 1 to 14, further comprising conventional additives and adjuvants.

16. The composition according to any one of claims 1 to 15, in which the active ingredient is mesalazine.

17. A foam comprising, per 100 parts by weight of foam:

   from 80 to 95 parts of the composition according to any one of claims 1 to 17; and
   from 5 to 25 parts of a propellent gas.

**18.** The foam according to claim 17, for rectal application.

**19.** A process for preparing a composition according to any one of claims 1 to 16, comprising the step of dispersing the powder of the active ingredient in water.

**20.** The process according to claim 19, comprising steps consisting of:

(i) preparing a buffer;
(ii) adding and mixing a muco-adhesive agent if present;
(iii) adding and mixing conventional additives, if present;
(iv) in parallel with the preceding steps, preparing a surfactant;
(v) mixing the product of step (iii) and the product of step (iv);
(vi) adding and dispersing the powder of the active ingredient.

**21.** The process according to claim 20, in which the surfactant is prepared by mixing two surfactants, one being a hydrophilic surfactant having an HLB value above 10, the other being a polyoxyalkylene.

**Patentansprüche**

**1.** Zusammensetzung eines Schaumes, der gewichtsmäßig, bezogen auf das Gesamtgewicht der Zusammensetzung,

(a) mehr als 25% eines Wirkstoffes in Pulverform,
(b) zwischen 1 und 20% eines Tensides enthält und
(c) dessen Rest aus Wasser zusammengesetzt ist;

dadurch gekennzeichnet,
daß das Pulver des Wirkstoffs eine Partikelgröße von weniger als 20 $\mu$m und eine Schüttdichte zwischen 250 und 450 g/dm$^3$ besitzt.

**2.** Zusammensetzung nach Anspruch 1, dadurch gekennzeichnet, daß die Partikelgröße weniger als 10 $\mu$m beträgt.

**3.** Zusammensetzung nach Anspruch 1 oder 2, dadurch gekennzeichnet, daß der Wirkstoff in Pulverform eine Schüttdichte zwischen 300 und 350 g/dm$^3$ besitzt.

**4.** Zusammensetzung nach linem der Anspruche 1 bis 3, dadurch gekennzeichnet, daß der Wirkstoff 30 bis 50% des Gewichtes ausmacht.

**5.** Zusammensetzung nach Anspruch 4, dadurch gekennzeichnet, daß der Wirkstoff 35 bis 45% des Gewichtes ausmacht.

**6.** Zusammensetzung nach einem der Ansprüche 1 bis 5, dadurch gekennzeichnet, daß das Tensid einen Anteil von 5 bis 10% darstellt.

**7.** Zusammensetzung nach einem der Ansprüche 1 bis 6, dadurch gekennzeichnet, daß das lensid eine Mischung aus zwei Tensiden ist, wobei es sich bei dem einen um ein hydrophiles Tensid mit einem HLB-Wert über 10 und bei dem anderen um ein Polyoxyalkylen handelt.

**8.** Zusammensetzung nach Anspruch 7, dadurch gekennzeichnet, daß das hydrophile Tensid einen HLB-Wert über 12 aufweist.

**9.** Zusammensetzung nach einem der Ansprüche 7 oder 8, dadurch gekennzeichnet, daß es sich bei dem hydrophilen Tensid um Polysorbat handelt.

**10.** Zusammensetzung nach einem der Ansprüche 8 bis 9, dadurch gekennzeichnet, daß es sich bei dem Tensid auf Polyoxyalkylen-Basis um ein Poloxamer handelt.

**11.** Zusammensetzung nach einem der Ansprüche 7 bis 10, dadurch gekennzeichnet, daß das Tensid auf Polyoxyalkylen-Basis ein Molekulargewicht von mehr als 5000 aufweist.

12. Zusammensetzung nach einem der Ansprüche 7 bis 11, dadurch gekennzeichnet, daß das Gewichtsverhältnis zwischen dem hydrophilen Tensid und dem Polyoxyalkylen zwischen 1 und 5 beträgt.

13. Zusammensetzung nach einem der Ansprüche 1 bis 12, dadurch gekennzeichnet, daß es sich bei dem Tensid um ein nicht-ionisches Tensid handelt.

14. Zusammensetzung nach einem der Ansprüche 1 bis 13, dadurch gekennzeichnet, daß sie außerdem ein muco-adhäsives Agens enthält.

15. Zusammensetzung nach einem der Ansprüche 1 bis 14, dadurch gekennzeichnet, daß die außerdem klassische Additive und Zusatzstoffe enthält.

16. Zusammensetzung nach einem der Ansprüche 1 bis 15, dadurch gekennzeichnet, daß es sich bei dem Wirkstoff um Mesalazin handelt.

17. Schaum, der je 100 Gewichtsanteilen :

 80 bis 95 Anteile der Zusammensetzung nach einem der Ansprüche 1 bis 16 sowie
 5 bis 25 Anteile Treibgas enthält.

18. Schaum nach Anspruch 17, für eine rektale Anwendung.

19. Verfahren zur Herstellung der Zusammensetzung nach einem der Ansprüche 1 bis 16, mit dem Schritt des Dispergieren des Wirkstoffpulvers in Wasser.

20. Verfahren nach Anspruch 19, umfassend die folgende Schritte:

 (i) Herstellen eines Puffers oder Tampons;
 (ii) Hinzufügen und Mischen des muco-adhäsiven Wirkstoffes, falls vorhanden;
 (iii) Hinzufügen und Mischen der klassischen Zusatzstoffe, falls vorhanden;
 (iv) Parallel zu den vorangehenden Schritten Herstellung des Tensides;
 (v) Mischen der in Schritt (iii) und (iv) erhaltenen Produkte;
 (vi) Hinzufügen und Dispersion des Wirkstoffpulvers.

21. Verfahren nach Anspruch 20, dadurch gekennzeichnet, daß das Tensid durch Mischen zweier Tenside hergestellt wird, wobei es sich bei dem einen um ein hydrophiles Tensid mit einem HLB-Wert von mehr als 10 und bei dem anderen um ein Polyoxyalkylen handelt.